# EUROPÄISCHE PATENTANMELDUNG

(11) **EP 1 872 770 A2**
(43) Veröffentlichungstag der Anmeldung: **02.01.2008**
(21) Anmeldenummer: 07110092.9
(22) Anmeldetag: 12.06.2007
(51) Int. Cl.: A61K 8/34, A61K 8/81, A61Q 17/04, A61Q 19/00

(54) **Kosmetische Zubereitung mit besonderer Textur**

(30) Priorität: 26.06.2006 DE 102006029559
(71) Anmelder: Beiersdorf AG, 20253 Hamburg (DE)
(72) Erfinder: Meiring, Uta, 21077, Hamburg (DE); Bleckmann, Andreas, 22926, Ahrensburg (DE); Behrens, Svea, 21109, Hamburg (DE)

(57) **Zusammenfassung**

Kosmetische Zubereitung, dadurch gekennzeichnet, dass sie
a) 0,1 bis 1 Gew.-% 1,2-Decandiol und
b) 0,1 bis 0,5 Gew.-% eines oder mehrerer Acrylat-Crosspolymere enthält.

## Beschreibung

Die vorliegende Erfindung betrifft kosmetische Zubereitungen - insbesondere kosmetische Zubereitungen vom Typ Öl- in- Wasser - mit einer besonderen Textur.

Unter Kosmetik kann man alle Maßnahmen zusammenfassen, die aus ästhetischen Gründen Veränderungen an Haut und Haaren vornehmen oder zur Körperreinigung angewendet werden. Kosmetik bedeutet also, das Körperäußere zu pflegen, zu verbessern und zu verschönern, um auf sichtbare, fühlbare und riechbare Weise sowohl den Mitmenschen als auch sich selbst zu gefallen. Schon vor Jahrtausenden wurde Kosmetik vom Menschen zu diesem Zweck angewandt. Man färbte Lippen und Gesicht, salbte sich mit wertvollen Ölen und badete in duftendem Wasser.

Während die Reinigungswirkung eines Shampoos, die Kämmbarkeitsverbesserung einer Haarspülung oder die Kräuselung des Haares durch eine Dauerwelle einfach und objektiv feststellbar sind, sind andere Wirkungen und Eigenschaften kosmetischer Produkte praktisch nicht messbar oder nur sehr individuell spürbar. Hierzu zählen zum Beispiel ein bestimmtes (belebendes, weiches, geschmeidiges, glattes etc.) Hautgefühl oder die Weichheit und Geschmeidigkeit der Haut nach der Anwendung eines Kosmetikums sowie die Fülle und Sprungkraft der Haare und dergleichen mehr. Ferner unterliegt die Erwartung der Verbraucher auch nebengeordneten Eigenschaften des Produktes. Hier sind insbesondere der Duft und die Farbe des Kosmetikums sowie seine Verpackung, der Preis, der Hersteller und die Werbeaussage zu nennen.

Eine für den Verbraucher sehr wesentliche, dabei aber nur schwer quantitativ messbare Eigenschaft kosmetischer Produkte ist ihre Textur. Unter dem Begriff "Textur" werden diejenigen Eigenschaften eines Kosmetikums verstanden, die auf den Gefügebau der Zubereitung zurückgehen, durch Tast- und Berührungssinne empfunden und ggf. in mechanischen oder rheologischen Fließeigenschaften ausgedrückt werden können. Die Textur kann insbesondere mittels Sensorik getestet werden. Die gegebenenfalls mit Hilfe von Zusatzstoffen beeinflussbare Textur kosmetischer Produkte ist für den Verbraucher von nahezu gleicher Bedeutung wie deren objektiv feststellbaren Wirkungen.

Mit dem Begriff "Sensorik" wird die wissenschaftliche Disziplin bezeichnet, die sich mit der Bewertung von kosmetischen Zubereitungen auf Grund von Sinneseindrücken befasst. Die sensorische Beurteilung eines Kosmetikums erfolgt anhand der visuellen, olfaktorischen und haptischen Eindrücke.
- *Visuelle Eindrücke:* alle mit dem Auge wahrnehmbaren Merkmale (Farbe, Form, Struktur).
- *Olfaktorische Eindrücke:* alle beim Einziehen von Luft durch die Nase wahrnehmbaren Geruchseindrücke, die häufig in Anfangsgeruch (Kopfnote), Hauptgeruch (Mittelnote, Körper) und Nachgeruch (Ausklang) differenziert werden können. Auch die erst bei der Anwendung freigesetzten flüchtigen Stoffe tragen zum olfaktorischen Eindruck bei.
- *Haptische Eindrücke:* alle Empfindungen des Tastsinns, die vornehmlich Gefüge und Konsistenz des Produktes betreffen.

Die sensorische Analyse macht von der Möglichkeit Gebrauch, den sensorischen Gesamteindruck eines Produktes integral zu erfassen. Nachteile der sensorische Analyse sind die Subjektivität des Eindrucks, eine leichte Beeinflussbarkeit der Prüfpersonen und die dadurch bedingte starke Streuung der Ergebnisse. Diesen Schwächen begegnet man heute durch den Einsatz von Gruppen geschulter Prüfpersonen, gegenseitige Abschirmung der Prüfer sowie statistische Auswertung der meist zahlreichen Analysendaten.

Die am häufigsten in der Forschung und Entwicklung genutzte Methode der sensorischen Analyse ist die Unterschiedsprüfung. Die Aufgabe beschränkt sich hierbei üblicherweise auf die Erkennung eines von mehreren Proben oder von einer Kontrollprobe abweichenden Musters. Während bei Unterschiedsprüfungen innerhalb eines Tests nur zwei Proben miteinander verglichen werden, ist bei der Rangordnungsprüfung eine Reihenfolge von drei und mehr Proben festzulegen und zwar in der Regel nach Intensität, Qualität, Beliebtheit oder Ähnlichkeit mit einer Vergleichsprobe. Diese (einfache) Methode eignet sich z. B. für eine Vorauswahl von Proben in der Produkt-Optimierung und wird auch häufig in der Marktforschung eingesetzt.

Die Textur einer kosmetischen Zubereitung lässt sich hilfsweise auch anhand ihrer rheologischen Fließeigenschaften charakterisieren.

Unter dem Begriff "Viskosität" versteht man die Eigenschaft einer Flüssigkeit, der gegenseitigen laminaren Verschiebung zweier benachbarter Schichten einen Widerstand (Zähigkeit, innere Reibung) entgegenzusetzen. Man definiert diese so genannte dynamische Viskosität nach η = τ/D als das Verhältnis der Schubspannung zum Geschwindigkeitsgradienten senkrecht zur Strömungsrichtung.

Während die graphische Darstellung des Fließverhaltens Newtonscher Flüssigkeiten bei konstanter Temperatur eine Gerade ergibt, zeigen sich bei den so genannten nicht newtonschen Flüssigkeiten in Abhängigkeit von der jeweiligen Schubspannung τ oft erhebliche Abweichungen. In diesen Fällen lässt sich die so genannte scheinbare Viskosität bestimmen, die zwar nicht der Newtonschen Gleichung gehorcht, aus der sich jedoch durch graphische Verfahren die wahren Viskositätswerte ermitteln lassen.

Unter dem Begriff Fließgrenze wird die kleinste Schubspannung verstanden, oberhalb derer ein plastischer Stoff sich rheologisch wie eine Flüssigkeit verhält (DIN 1342-1: 1983-10). Die Bestimmung der Fließgrenze erfolgt durch Aufnahme einer Fließkurve (DIN 53019: 1980-05; DIN 53214: 1982-02). Der erhaltene Wert hängt stark von der Zeitskala (Belastungsrate) ab, die der Messung zugrunde liegt. Die Fließkurve ist die graphische Darstellung des Zusammenhangs zwischen Schubspannung und Geschwindigkeitsgefälle D für eine einer Schichtenströmung unterworfenen Flüssigkeit oder für einen plastischen Stoff oberhalb der Fließgrenze. Die Messung von Fließkurven erfolgt üblicherweise in Rotationsviskosimetern. Bei drehzahlgesteuerten Systemen wird unter Vorgabe eines kontinuierlich oder schrittweise variierten Geschwindigkeitsgefälles das resultierende Drehmoment gemessen und die dazu proportionale Schubspannung errechnet. Bei schubspannungskontrollierten Systemen gilt der umgekehrte Sachverhalt.

Aus der Fließkurve können die Viskosität als Funktion des Geschwindigkeitsgefälles errechnet, Fließgrenzen bestimmt und das Fließverhalten charakterisiert werden.

Kosmetische Formulierungen weisen häufig ein pseudoplastisches Fließverhalten mit mehr oder minder ausgeprägter Thixotropie auf, die es dem Verbraucher erleichtert, solche Produkte zu applizieren. Außerdem bestimmen die Stärke und Art der Pseudoplastizität sowie die Thixotropie auch die Verteilung auf der Haut und das Hautgefühl nach dem Eincremen, weil beispielsweise eine thixotrope O/W-Formulierung nach dem Verteilen wieder eine (fühlbare) Struktur auf der Haut aufbauen kann.

Natürlich ist dem Fachmann eine Vielzahl von Möglichkeiten bekannt, stabile Zubereitungen zur kosmetischen Anwendung zu formulieren, beispielsweise in Form von Cremes und Salben, die im Bereich von Raum- bis Hauttemperatur streichfähig sind, oder als Lotionen und Milche, die in diesem Temperaturbereich eher fließfähig sind.

Kosmetische Zubereitungen zur (großflächigen) Anwendung im Körperpflegebereich - d. h. Zubereitungen, die insbesondere in relativ großer Menge aus üblichen Kunststoffflaschen entnehmbar sein sollen - werden in der Regel so formuliert, dass sich eine leichte Verteilbarkeit auf der Haut und gleichzeitig eine gute Restentleerung aus der Verpackung einstellt. Um beiden Kriterien gerecht werden zu können, müssen die Zubereitungen des Standes der Technik dementsprechend eine eher niedrige Viskosität von max. 6000 mPa·s (bestimmbar mit einem Haake Viskotester VT-02 bei 25 °C) haben. Diese Anforderung an ein entsprechendes Hautpflegeprodukt führt dementsprechend zu immer gleichen bzw. ähnlichen Rezepturen.

Eine Aufgabe der vorliegenden Erfindung war es daher, Zubereitungen zu finden, welche neben den für Kosmetika üblichen Kriterien wie Verträglichkeit, Lagerstabilität und dergleichen auch für den Verbraucher wesentliche, bisher nicht gekannte kosmetische Leistungen bieten. Insbesondere sollten sich die gesuchten Zubereitungen für eine Verwendung im Körperpflegebereich, d. h. bei einer Anwendung auf dem ganzen Körper (und damit in Abgrenzung von einer reinen Gesichtspflegeanwendung in relativ großer Menge) eignen, dabei aber gleichzeitig für eine Anwendung im Gesichtsbereich sensorisch attraktiv sein. Dies äußert sich insbesondere in einer guten Verteilbarkeit, einem schnellen Einzugsvermögen sowie in einer gehaltvollen und pflegenden Anmutung.
Überraschend hat sich gezeigt, dass eine
kosmetische Zubereitung, dadurch gekennzeichnet, dass sie
a) 0,1 bis 1 Gew.-% 1,2-Decandiol und
b) 0,1 bis 0,5 Gew.-% eines oder mehrerer Acrylat-Crosspolymere enthält,
diese Aufgaben zu lösen vermögen.

Die erfindungsgemäßen Zubereitungen stellen in jeglicher Hinsicht überaus befriedigende Präparate dar. Es war für den Fachmann nicht vorauszusehen gewesen, dass sich Emulsionen, welche durch die gezielte Einstellung des pseudoplastischen Fließverhaltens (Strukturviskosität) und der Thixotropie die erfindungsgemäßen Eigenschaften zeigen, trotz relativ hoher Fließgrenze insbesondere sehr leicht auf der Haut verteilen lassen, schnell einziehen und dennoch sehr gehaltvoll und pflegend sind.

Es ist bevorzugt im Sinne der vorliegenden Erfindung, den Gehalt an 1,2-Decandiol aus dem Bereich von 0,3 bis 0,7 Gew.-%, besonders bevorzugt wie ca. 0,5 Gew.-% zu wählen, jeweils bezogen auf das Gesamtgewicht der Zubereitung.

Es kann erfindungsgemäß vorteilhaft sein, 1,2-Decandiol mit anderen 1,2-Alkandiolen zu kombinieren und so die die erfindungsgemäßen Eigenschaften weiter zu verbessern. Besonders vorteilhaft sind Kombinationen mit 1,2-Hexandiol und/oder 1,2-Octandiol.

In diesem Fall ist es vorteilhaft im Sinne der vorliegenden Erfindung, das Mengenverhältnis von 1,2-Decandiol zu den anderen 1,2-Alkandiolen (insbesondere 1,2-Hexandiol und/oder 1,2-Octandiol) wie 3 : 1 bis 1 : 3, besonders vorteilhaft wie 1 : 1 zu wählen.

Es ist bevorzugt im Sinne der vorliegenden Erfindung, den Gehalt an allen 1,2-Alkandiolen aus dem Bereich von 0,5 bis 2 Gew.-%, besonders bevorzugt aus dem Bereich von 0,75 bis 1,5 Gew.-% zu wählen, jeweils bezogen auf das Gesamtgewicht der Zubereitung.

Acrylat-Crosspolymere im Sinne der vorliegenden Erfindung sind Polymere (Makromoleküle) mit hohem Molekulargewicht (> 1 Mg/mol), welche aus einem Gerüst aus Polyacrylsäure und geringen Mengen an Polyalkenyl-Polyether-Quervernetzungen bestehen. Sie werden auch als Carbomer bezeichnet. Diese wasserlöslichen oder dispergierbaren Polymere können in der Flüssigkeit, in der sie gelöst oder dispergiert sind, eine bedeutende Viskositätserhöhung hervorrufen. Dies wird durch die Bildung von Carbomer-Mikrogelen im Wasser hervorgerufen.

Besonders bevorzugte Acrylat-Crosspolymere im Sinne der vorliegenden Erfindung sind polymere Emulgatoren. Polymere Emulgatoren sind hauptsächlich Polyacrylsäurepolymere mit hohem Molekulargewicht. Diese Emulgatoren haben einen kleinen lipophilen Anteil zusätzlich zum hydrophilen Hauptteil. Dadurch wirken sie primär als Emulgatoren, aber gleichzeitig auch als Öl-in-Wasser (O/W-) Emulsionsstabilisatoren. Der lipophile Anteil adsorbiert an die Ölphase und das hydrophile Gerüst quillt in der Wasserphase unter Ausbildung einer Gelstruktur um die Öltröpfchen herum, einhergehend kommt es zur Stabilisierung der Emulsion.

Ganz besonders bevorzugt im Sinne der vorliegenden Erfindung sind Acrylat-Crosspolymere, welche die INCI-Bezeichnung "Acrylates/C10-30 Alkyl Acrylate Crosspolymer" haben und unter den Handelsbezeichnungen Pemulen TR-1 und Pemulen TR-2 sowie Carbopol 1342, Carbopol 1382 und Carbopol ETD 2020 von der Firma NOVEON erhältlich sind.

Es ist bevorzugt im Sinne der vorliegenden Erfindung, den Gehalt an Acrylat-Crosspolymeren (eine oder mehrere Verbindungen) aus dem Bereich von 0,1 bis 1,5 Gew.-%, besonders bevorzugt 0,15 bis 1 %, ganz besonders bevorzugt 0,15 bis 0,75 Gew.-% zu wählen, jeweils bezogen auf das Gesamtgewicht der Zubereitung.

Die Textur der erfindungsgemäßen kosmetischen Zubereitungen lässt sich hilfsweise anhand ihrer rheologischen Fließeigenschaften charakterisieren. Dazu werden Fließkurven der Zubereitungen mit einem SR-2000 der Fa. Rheometric Scientific (jetzt Fa. TA-Instruments) aufgezeichnet. Dieses Gerät ist ein schubspannungsgesteuertes Rheometer mit einem luftgelagerten Transducer. Das Meßsystem besteht aus einem parallelen Plattenmeßsystem (sog. Platte/Platte-Anordnung) mit einem Durchmesser von 25 mm, wobei die untere Platte mit einem Peltierelement temperierbar ist. Die Messung wird bei einer Meßtemperatur von 25 °C durchgeführt. Als Meßmethode wird eine lineare Schubspannungs-Zeit-Rampe mit einer Belastungsrate von 40 Pa/min beginnend bei 0 Pa gewählt.
Die erfindungsgemäßen Zubereitungen stellen daher eine erhebliche Bereicherung des Standes der Technik in bezug auf fließfähige Zubereitungen dar.

Die erfindungsgemäßen kosmetischen Zubereitungen können wie üblich zusammengesetzt sein und zur Behandlung und/oder Pflege der Haut und als Schminkprodukt in der dekorativen Kosmetik dienen.

Besonders bevorzugt stellen die erfindungsgemäßen Zubereitungen Emulsionen oder Hydrodispersionen dar. Im Gegensatz zur klassischen Emulsionen enthalten Hydrodispersionen aber nur sehr geringe Mengen an Emulgatoren (bis zu 2 Gew.-%) bzw. können sogar gänzlich frei von Emulgatoren sein.

Bei Hydrodispersionen, welche aus einer flüssigen Ölphase in einer äußeren wässrigen Phase bestehen, wird die Stabilität des Mehrphasensystems üblicherweise dadurch gewährleistet, dass in der wässrigen Phase mit Hilfe eines Gelbildners ein Gelgerüst aufgebaut wird, in welchem die Lipidtröpfchen stabil suspendiert sind. Besonders bevorzugt ist es, wenn die erfindungsgemäßen Hydrodispersionen frei von Emulgatoren sind.

Die Ölphase der erfindungsgemäßen Zubereitungen kann folgende Bestandteile enthalten.

Diese werden vorteilhaft gewählt aus der Gruppe der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Besonders vorteilhaft im Sinne der vorliegenden Erfindung ist Capryl-Caprinsäuretriglycerid (INCI: Caprylic/capric Triglyceride) zu verwenden.

Die Fettsäuretriglyceride können vorteilhaft gewählt werden aus der Gruppe der synthetischen, halbsynthetischen und natürlichen Öle, wie z. B. Olivenöl, Sonnenblumenöl, Sojaöl, Erdnussöl, Rapsöl, Mandelöl, Palmöl, Kokosöl, Rizinusöl, Weizenkeimöl, Traubenkernöl, Distelöl, Nachtkerzenöl und dergleichen mehr.

Die Ölphase kann im Sinne der vorliegenden Erfindung ferner vorteilhaft Substanzen aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen enthalten. Solche Esteröle können dann vorteilhaft gewählt werden aus der Gruppe Isopropylpalmitat, Isopropylstearat, n-Butylstearat, n-Hexyllaurat, Isooctylstearat, Isononylstearat, Isononylisononanoat, 2-Ethylhexylpalmitat, 2-Hexyldecylstearat, 2-Octyldodecylpalmitat, Erucylerucat sowie synthetische, halbsynthetische und natürliche Gemische solcher Ester, wie z. B. Jojobaöl.

Es ist ferner vorteilhaft im Sinne der vorliegenden Erfindung, wenn die Zubereitungen ein oder mehrere flüchtige Öle enthalten, z. B. aus der Gruppe Phenyltrimethicon (INCI-Bezeichnung: Phenyl Trimethicone, CAS 2116-84-9), Cyclomethicone (INCI-Bezeichnung: Cyclomethicone, CAS 556-67-2 u. 69430-24-6) - insbesondere Octamethylcyclotetrasiloxan, Cyclomethicone D5 und/oder Cyclomethicone D6 - und C12-20 Isoparaffin bzw. Isohexadekan.

Die Bestandteile der Ölphase können beliebige Abmischungen von Öl-, Fett- und/oder Wachskomponenten darstellen.

Ferner ist es vorteilhaft im Sinne der vorliegenden Erfindung, wenn die Zubereitungen einen oder mehrere Fettalkohole enthalten. Bevorzugter Fettalkohol ist der Cetylalkohol.

Die Gesamtmenge an einem oder mehreren Fettalkoholen in den fertigen kosmetischen Zubereitungen wird vorteilhaft aus dem Bereich von 0,1 bis 10,0 Gew.-%, bevorzugt 0,5 bis 6,0 Gew.-% gewählt, jeweils bezogen auf das Gesamtgewicht der Zubereitungen.

Die erfindungsgemäße kosmetische Zubereitung kann erfindungsgemäß vorteilhaft in Form einer Wasser-in-Öl-Emulsion (W/O-Emulsion) vorliegen. In diesem Fall sind die folgende Emulgatoren erfindungsgemäß bevorzugt:

| **Handelsname** | **INCI-Name** |
|---|---|
| Lameform TGI | Polyglyceryl-3 Diisostearate |
| Isolan Gl 34 | Polyglyceryl-4 Isostearate |
| Dehymuls PGPH | Polyglyceryl-2 Dipolyhydroxystearate |
| Arlacel P 135 | PEG-30 Dipolyhydroxystearate |
| Eucerit PA | Lanolin Alcohol |
| Atlas G-1 049 | PEG-40 Sorbitan Perisostearate |
| Abil EM 90 | Cetyl Dimethicone Copolyol |
| Arlacel 989 | PEG-7 Hydrogenated Castor Oil |
| Elfacos ST 9 | PEG 45/Dodeceyl Glycol Copolymer |
| Elfacos ST 37 | PEG 22/Dodeceyl Glycol Copolymer |
| Isostearinsäure PP | Pentaerythrityl Isostearate |
| Imwitor 780 K | Isostearyl Diglyceryl Succinate |
| Arlacel 987 | Sorbitan Isostearate |
| Hostacerin DGI | Polyglyceryl-2 Sesquiisostearate |
| Tegin ISO | Glyceryl Isostearate |
| Arlacel 60 | Sorbitan Stearate |
| Tegin M | Glyceryl Stearate |
| Arlantone G | PEG-25 Hydrogenated Castor Oil |
| Arlantone T | PEG-40 Sorbitan Peroleate |
| Arlacel 80 | Sorbitan Oleate |
| | Cera Microcristallina + Paraffinum Liquidum + Ozokerite+ Hydrogenated Castor Oil + Glyceryl Isostearate + Polyglyceryl-3 Oleate |
| Atlas G-1 049 | PEG-40 Sorbitan Perisostearate |
| ABIL WS 08 | Cetyl Dimethicone Copolyol + Hexyl Laurate + Polyglyceryl-3 Oleate + Cetyl Dimethicone |
| Hostacerin DGO | Polyglyceryl-2 Sesquioleate |
| Abil WE 09 | Cetyl Dimethicone Copolyol (+) Polyglyceryl-4 Isostearate (+) Hexyl Laurate |
| Abil EM 90 | Cetyl PEG/ PPG- 10/1- Dimethicone |
| Abil EM 97 | Dimethicone Copolyol (+) Cyclomethicone |
| Isolan GO 33 | Polyglyceryl-3 Oleate |
| Montanov WO 18 | Isostearyl Alcohol (+) Isostearyl Glucoside |
| Cremophor GO 32 | Polyglyceryl-3 Dioleate |
| Olivem 900 | Sorbitan Oleate |
| Sisterna SP01-C | Sucrose Distearate |
| Sisterna SP10-C | Sucrose Distearate |
| Dehymuls B | Polyglyceryl-3 Diisostearate (+) Glyceryl Oleate |
| Emulsogen SRH | Rapeseed Sorbitols |
| Emulsogen SRO | Rapeseed Sorbitols |
| Rylo PG 11 | Polyglyceryl Dimer Soyate |
| Grindstedt PS 401 (Dermofeel PR) | Polyglyceryl Polyricinoleate |
| Isolan PDI | Polyglyceryl-3 Dimerate |
| Arlacel 986 | Glyceryl Sorbitan Stearate |
| Decaglyn 5-HS | Polyglyceryl-10 Hydroxystearate |

Ganz besonders bevorzugt ist es, wenn der oder die W/O-Emulgatoren gewählt werden aus der Gruppe PEG-30 Dipolyhydroxystearat, Polyglyceryl-3-Diisostearat, PEG-40 Sorbitan Perisostearate.

In einer zweiten erfindungsgemäß vorteilhaften Ausführungsform der vorliegenden Erfindung kann die erfindungsgemäße kosmetische Zubereitung in Form einer Öl-in-Wasser-Emulsion (OM/-Emulsion) vorliegen.

In diesem Fall werden der oder die O/W-Emulgatoren erfindungsgemäß vorzugsweise aus der folgenden Gruppe gewählt:
Glycerylstearat in Kombination mit Ceteareth-20 und/oder Ceteareth-25, Ceteareth-6 in Kombination mit Stearylalkohol, Cetylstearylalkohol in Kombination mit PEG-40-Ricinusöl und Natriumcetylstearylsulfat, Triceteareth-4 Phosphat, Glycerylstearat, Natriumcetylstearylsulfat, Lecithin Trilaureth-4 Phosphat, Laureth-4 Phosphat, Stearinsäure, Propylenglycolstearat SE, PEG-25-hydriertes Ricinusöl, PEG-54-hydriertes Ricinusöl und/oder PEG-6 Caprylsäure/Caprinsäureglyceride, Glyceryloleat in Kombination mit Propylenglycol, PEG-9-Stearat, PEG-20 Stearat, PEG-30-Stearat, PEG-40-Stearat, PEG-100-Stearat, Ceteth-2, Ceteth-20, Polysorbate-20, Polysorbate-60, Polysorbate-65 und/oder Polysorbate-100, Glycerylstearat in Kombination mit PEG-100 Stearat, Glycerylmyristat, Glyceryllaurat, PEG-40-Sorbitanperoleat, Laureth-4, Ceteareth-3 und/oder Isostearylglycerylether, Cetylstearylalkohol in Kombination mit Natrium Cetylstearylsulfat, Laureth-23 und/oder Steareth-2, Glycerylstearat in Kombination mit PEG-30 Stearat, PEG-40-Stearat, Glycol Distearat, PEG-22-Dodecyl Glycol Copolymer, Polyglyceryl-2-PEG-4-Stearat, Ceteareth-12, Ceteareth-20, Ceteareth-30, Methyl-glucosesesquistearat, Steareth-10 und/oder PEG-20-Stearat, Steareth-2 in Kombination mit PEG-8 Distearat, Steareth-21, Steareth-20, Isosteareth-20, PEG-45/ Dodecylglycol-Copolymer, Methoxy-PEG-22/Dodecylglycol-Copolymer, PEG-40-Sorbitanperoleat, PEG-40-Sorbitanperisostearat, PEG-20-Glycerylstearat, PEG-20-Glycerylstearat, PEG-8-Bienenwachs, Polyglyceryl-2-laurat, Isostearyldiglycerylsuccinat, Stearamidopropyl-PG-dimoniumchloridphosphat, Glycerylstearat SE, Ceteth-20, Triethylcitrat, PEG-20-Methylglucosesesquistearat, Glycerylstearatcitrat, Cetylphosphat, Cetearyl Sulfat, Sorbitansesquioleat, Triceteareth-4-Phosphat, Trilaureth-4-Phosphat, Polyglyceryl-methylglucosedistearat, Kaliumcetylphosphat, Polyglyceryl-3 Methylglucose Distearate Isosteareth-10, Polyglyceryl-2-sesquiiostearat, Ceteth-10, Oleth-20 und/oder Isoceteth-20, Glycerylstearat in Kombination mit Ceteareth-20, Ceteareth-12, Cetylstearylalkohol und/oder Cetylpalmitat, Cetylstearylalkohol in Kombination mit PEG-20 Stearat, PEG-30-Stearat, PEG-40-Stearat und/oder PEG-100-Stearat.

Als erfindungsgemäß besonders vorteilhafte O/W-Emulgatoren werden Glycerylstearat, PEG-40 Stearat, Triglycerinmethylglucosedistearat, Polyglyceryl-3 Methylglucose Distearate, Polyethylenglycol(21)stearylether, Polyethylenglycol(2)stearylether, Cetearylglucosid, Glycerylstearatcitrat, Natriumcetearyl-sulfat, Cetearylalkohol und/oder Sorbitanstearat eingesetzt.

In einer dritten erfindungsgemäß vorteilhaften Ausführungsform der vorliegenden Erfindung kann die erfindungsgemäße kosmetische Zubereitung in Form eines Gels vorliegen.

Im technischen Sinn werden unter dem Begriff "Gel" relativ formbeständige, leicht verformbare disperse Systeme aus zumindest zwei Komponenten verstanden, welche in der Regel aus einem - meist festen - kolloid zerteilten Stoff aus langkettigen Molekülgruppierungen (z. B. Gelatine, Kieselsäure, Polysaccharide) als Gerüstbildner und einem flüssigen Dispersionsmittel (z. B. Wasser) bestehen. Der kolloidal zerteilte Stoff wird oft als Verdickungsmittel, Gelbildner oder Geliermittel bezeichnet. Er bildet ein räumliches Netzwerk im Dispersionsmittel, wobei einzelne kolloidal vorliegende Partikel über elektrostatische Wechselwirkung miteinander mehr oder weniger fest verknüpft sein können.

Das Dispersionsmittel, welches das Netzwerk umgibt, zeichnet sich durch elektrostatische Affinität zum Verdickungsmittel aus, d. h., ein vorwiegend polares (insbesondere: hydrophiles) Verdickungsmittel geliert vorzugsweise ein polares Dispersionsmittel (insbesondere: Wasser).

Besonders bevorzugt stellen die erfindungsgemäßen Zubereitungen transparente Gele dar. In einer besonders bevorzugten Ausführungsform enthalten die Zubereitungen im Sinne der vorliegenden Erfindung keinerlei Ölkomponenten.

Als transparent im Sinne der vorliegenden Erfindung werden Gele bezeichnet, welche in einer Schichtdicke von 1 cm bei einer Transmissionsmessung mit einem UV/VIS-Spektrometer bei einer Wellenlänge von 600 nm eine Transmission von mehr als 90 % zeigen.

Als transluzent im Sinne der vorliegenden Erfindung werden Gele bezeichnet, welche in einer Schichtdicke von 1 cm bei einer Transmissionsmessung mit einem UV/VIS-Spektrometer bei einer Wellenlänge von 600 nm eine Transmission von mehr als 60 % zeigen.

Gele im Sinne der vorliegenden Erfindung können neben den erfindungsgemäßen Acrylat-Crosspolymeren vorteilhaft ein oder mehrere Hydrokolloide als Verdickungsmittel enthalten.

Das oder die Hydrokolloide werden erfindungsgemäß vorteilhaft aus einer oder mehreren der folgenden Gruppen gewählt:
- organische, natürliche Verbindungen, wie beispielsweise Agar-Agar, Carrageen, Tragant, Gummi arabicum, Alginate, Pektine, Polyosen, Guar-Mehl, Johannisbrotbaumkernmehl, Stärke, Dextrine, Gelatine, Casein,
- organische, abgewandelte Naturstoffe, wie z. B. Carboxymethylcellulose und andere Celluloseether, Hydroxyethyl- und -propylcellulose und mikrokristalline Cellulose dergleichen,
- organische, vollsynthetische Verbindungen, wie z. B. Polyacryl- und Polymethacryl-Verbindungen, Vinylpolymere, Polycarbonsäuren, Polyether, Polyimine, Polyamide, Polyurethane.

Bevorzugt sind sowie insbesondere Polysaccharide bzw. deren Derivate - z. B. Hyaluronsäure, Xanthangummi, Hydroxypropylmethylcellulose, jeweils einzeln oder in Kombination.

Erfindungsgemäß besonders vorteilhafte Hydrokolloide sind ferner Verbindungen, die die INCI-Bezeichnung Ammonium Acryloyldimethyltaurate / VP Copolymer tragen.

Erfindungsgemäß vorteilhaft weisen diese Verbindungen die Summenformel [C₇H₁₆N₂SO₄]ₙ [C₆H₉NO]ₘ auf, einer statistischen Struktur wie folgt entsprechend

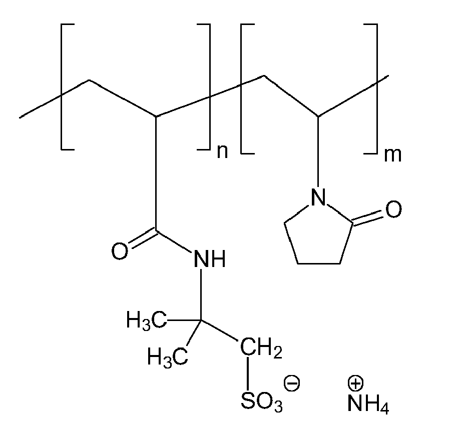

Bevorzugte Spezies im Sinne der vorliegenden Erfindung sind in den Chemical Abstracts unter den Registraturnummern 58374-69-9, 13162-05-5 und 88-12-0 abgelegt und erhältlich unter der Handelsbezeichnung Aristoflex® AVC der Gesellschaft Clariant GmbH.

Vorteilhaft sind ferner Copolymere/Crosspolymere umfassend Acryloyldimethyl Taurate, wie beispielsweise Simugel® EG oder Simugel® EG von der Gesellschaft Seppic S.A.

Zur Anwendung werden die erfindungsgemäßen Zubereitungen in der für Kosmetika üblichen Weise auf die Haut in ausreichender Menge aufgebracht.

Die kosmetischen Zubereitungen gemäß der Erfindung können ferner kosmetische Hilfsstoffe enthalten, wie sie üblicherweise in solchen Zubereitungen verwendet werden, z. B. Konservierungsmittel, Konservierungshelfer, Komplexbildner, Bakterizide, Parfüme, Substanzen zum Verhindern oder Steigern des Schäumens, Pigmente, die eine färbende Wirkung haben, Verdickungsmittel, anfeuchtende und/oder feuchthaltende Substanzen, Füllstoffe, die das Hautgefühl verbessern, oder andere übliche Bestandteile einer kosmetischen Formulierung wie Alkohole, Polyole, Polymere, Schaumstabilisatoren, Elektrolyte, organische Lösungsmittel oder Silikonderivate.

Geeignete Konservierungsmittel im Sinne der vorliegenden Erfindung sind beispielsweise Formaldehydabspalter (wie z. B. DMDM Hydantoin, welches beispielsweise unter der Handelsbezeichnung Glydant^{™} von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfasst das Konservierungssystem erfindungsgemäß ferner vorteilhaft auch Konservierungshelfer, wie beispielsweise Octoxyglycerin, Glycine Soja etc.

Obwohl die genannten Konservierungsmittel an sich in den erfindungsgemäßen Zubereitungen vorteilhaft eingesetzt werden können, sind ganz besonders bevorzugt solche Zubereitungen, welche frei von Parabenen und/oder frei von Formaldehydabspaltern sind.

Vorteilhafte Komplexbildner im Sinne der vorliegenden Erfindung sind beispielsweise EDTA, [S,S]-Ethylendiamindisuccinat (EDDS), welches beispielsweise unter der Handelsbezeichnung Octaquest von der Fa. Octel erhältlich ist, Pentanatrium-Ethylendiamintetramethylenphosphonat, welches z. B. unter dem Handelsnamen Dequest 2046 von der Fa. Monsanto erhältlich ist und/oder Iminodibersteinsäure, welche u. a. von der Fa. Bayer AG unter den Handelsnamen Iminodisuccinat VP OC 370 (ca. 30% ige Lösung) und Baypure CX 100 fest erhältlich ist.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetischen Zubereitungen gemäß der vorliegenden Erfindung kosmetische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Carnitin, Carnosin, natürliche und/oder synthetische Isoflavonoide, Kreatin, Taurin und/oder β-Alanin sowie 8-Hexadecen-1,16-dicarbonsäure (Dioic acid, CAS-Nummer 20701-68-2; vorläufige INCI-Bezeichnung Octadecendioic acid). Erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, eignen sich insbesondere vorteilhaft zur Prophylaxe und Behandlung kosmetischer Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen). Weiterhin vorteilhaft eignen sie sich gegen das Erscheinungsbild der trockenen bzw. rauen Haut.

Die Wasserphase der Zubereitungen gemäß der vorliegenden Erfindung kann vorteilhaft übliche kosmetische Hilfsstoffe enthalten, wie beispielsweise Alkohole, insbesondere solche niedriger C-Zahl, vorzugsweise Ethanol und/oder Isopropanol, Polymere, Schaumstabilisatoren und/oder Elektrolyte.

Ferner vorteilhaft können die Zubereitungen gemäß der vorliegenden Erfindung auch Repellentien zum Schutz vor Mücken, Zecken und Spinnen und dergleichen enthalten. Vorteilhaft sind z. B. N,N-Diethyl-3-methylbenzamid (Handelsbezeichnung: Metadelphene, "DEET"), Dimethylphtalat (Handelsbezeichnung: Palatinol M, DMP) sowie insbesondere 3-(N-n-Butyl-N-acetyl-amino)-propionsäureethylester (unter dem Handelsnamen Insekt Repellent® 3535 bei der Fa. Merck erhältlich). Ein weiterer vorteilhafter Repellent-Wirkstoff im Sinne der vorliegenden Erfindung ist der 1-Piperidincarboxylsäure 2-(2-hydroxyethyl)-1-methylpropylester (INN: Icaridin, CAS-Nummer: 119515-38-7, Elincs-Nummer: 423-210-8), der die folgende Struktur 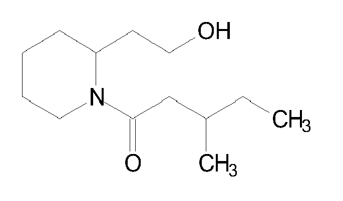 aufweist und als besonders wirksam gilt.
Die Repellentien können sowohl einzeln als auch in Kombination eingesetzt werden.
Auch sog. Moisturizer sind vorteilhaft zu verwenden. Als Moisturizer werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.
Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile^{®} (CAS-Nr. 7631-86-9).

In einer weiteren vorteilhaften Ausführungsform liegen die erfindungsgemäßen Zubereitungen in der Form eines Sonnenschutzmittels vor. Es ist aber auch vorteilhaft im Sinne der vorliegenden Erfindungen, solche Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescrèmes gewöhnlich UV-A- bzw. UV-B-Filtersubstanzen eingearbeitet.

Für den Fall, dass ein Gehalt an UV-Filtersubstanzen gewünscht ist, werden diese vorteilhaft aus einer oder mehreren der folgenden Gruppen gewählt: UV-A-, UV-B- und/oder Breitbandfiltersubstanzen sowie organische und/oder anorganische Pigmente als UV-Filtersubstanzen.

Besonders vorteilhafte UV-Filtersubstanzen im Sinne der vorliegenden Erfindung sind:
- Dibenzoylmethanderivate, insbesondere das 4-(tert.-Butyl)-4'-methoxydibenzoylmethan (CAS-Nr. 70356-09-1), welches von Givaudan unter der Marke Parsol^{®} 1789 und von Merck unter der Handelsbezeichnung Eusolex® 9020 verkauft wird.
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Disodium Phenyl Dibenzimidazol Tetrasulfonat (CAS-Nr.: 180898-37-7), welches beispielsweise unter der Handelsbezeichnung Neo Heliopan AP bei Symrise erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Symrise erhältlich ist;
- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornyliden-methyl)benzolsulfonsäure, 2-Methyl-5-(2-oxo-3-bornylidenmethyl)sulfonsäure und deren Salze.
- Benzoxazol-Derivate, wie z. B. das 2,4-bis-[5-1(dimethylpropyl)benzoxazol-2-yl-(4-phenyl)-imino]-6-(2-ethylhexyl)-imino-1,3,5-triazin mit der CAS Nr. 288254-16-0, welches bei 3V Sigma unter der Handelsbezeichnung Uvasorb® K2A erhältlich ist.
- Hydroxybenzophenone, z. B. der 2-(4'-Diethylamino-2'-hydoxybenzoyl)-benzoesäurehexylester (auch: Aminobenzophenon), welcher unter der Handelsbezeichnung Uvinul A Plus bei der Fa. BASF erhältlich ist.
- Triazinderivate, wie z. B. 2,4-Bis-{[4-(2-Ethyl-hexyloxy)-2-hydroxy]-phenyl}-6-(4-methoxyphenyl)-1,3,5-triazin (INCI: Bis-Ethylhexyloxylphenol Methoxyphenyl Triazin), welches unter der Handelsbezeichnung Tinosorb® S bei der CIBA-Chemikalien GmbH erhältlich ist; Dioctylbutylamidotriazon (INCI: Diethylhexyl Butamido Triazone), welches unter der Handelsbezeichnung UVASORB HEB bei Sigma 3V erhältlich ist; 4,4',4"-(1,3,5-Triazin-2,4,6-triyltriimino)-tris-benzoesäure-tris(2-ethylhexylester), auch: 2,4,6-Tris-[anilino-(p-carbo-2'-ethyl-1'-hexyloxy)]-1,3,5-triazin (INCI: Ethylhexyl Triazone), welches von der BASF Aktiengesellschaft unter der Warenbezeichnung UVI-NUL® T 150 vertrieben wird; 2-[4,6-Bis(2,4-dimethylphenyl)-1,3,5-triazin-2-yl]-5-(octyloxy)phenol (CAS Nr.: 2725-22-6).
- Benzotriazole, wie z. B. 2,2'-Methylen-bis-(6-(2H-benzotriazol-2-yl)-4-(1,1,3,3-tetramethylbutyl)-phenol) (INCI: Methylene Bis-Benztriazolyl Tetramethylbutylphenol), welches z. B. unter der Handelsbezeichnung Tinosorb® M bei der CIBA-Chemikalien GmbH erhältlich ist.
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon sowie
- an Polymere gebundene UV-Filter
- Ethylhexyl-2-cyano-3,3-diphenylacrylat (Octocrylen), welches von BASF unter der Bezeichnung Uvinul^{®} N 539 T erhältlich ist.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele:

| O/W -Lotion: | | | | | | | | |
|---|---|---|---|---|---|---|---|---|
| pH-Wert auf 5,5 bis 7 einstellen | | | | | | | | |
| | **1** | **2** | **3** | **4** | **5** | **6** | **7** | **8** |
| Glyceryl Stearate+ PEG-100 stearate | 1,3 | | | | | | | |
| Steareth-2 | 1,4 | | | 0,4 | | | | |
| Ceteareth-20 | 1 | 2 | | 1 | | | | |
| Glycerylstearate+PEG-30 stearate | | 1,5 | | | | | | |
| PEG-40 stearate | | | 2 | | | | | |
| Polysorbate 60 | | | 2,5 | | | | | |
| Glyceryl Stearate+PEG 100 stearate | | | | 1,25 | | | | |
| Glyceryl Stearate Citrate | | | | | | | 2 | |
| Dicaprylyl Carbonate | | | | | 3 | 4 | | 2 |
| Isodecyl Neopentanoate | | | | | 3 | 4 | | |
| Squalane | | | | | 3 | 2 | 2,5 | |
| Butyrospermum Parkii | | | | | 1 | 0,5 | | |
| C12-15 Alkyl Benzoate | 3,5 | 3 | | 5 | | | | 1 |
| Octyldodecanol | 3 | 4 | | 2 | | | | |
| Dimethicone | 1 | 2 | 3 | 0,5 | 2 | 2 | | 1 |
| Cyclomethicone | 6 | 4 | 3 | 5 | 7 | 6 | 2 | 4 |
| Paraffinum Liquidum | | | 3 | | | | | |
| Caprylic Capric Triglyceride | | | 2,5 | | | | | |
| Hydrogenated Poly-isobutene | | | | | | | 5 | |
| Hydrogenated Coco-Glycerides | | | 2,5 | | | | | |
| Cetearyl Alkohol | | | | | 0,5 | 0,75 | | |
| 1,2 Decandiol | 0,5 | 0,3 | 0,35 | 0,4 | 0,2 | 0,15 | 0,3 | 0,3 |
| Sensoric modifier (Talc, starch, etc) | 1 | 0,5 | | | | | | 3 |
| Butyl Methoxybenzoylmethane | 1 | 1 | | 1 2 | | | | |
| Ethylhexyl Methoxycinnamate | 3 | 4 | | 3 | | | | |
| Acrylates/C10-30 Alkyl Acrylate Crosspolymer | 0,15 | 0,15 | 0,25 | 0,15 | 0,1 | 0,1 | 0,15 | 0,3 |
| Ammonium Acryloyl-dimethyltaurate/VP Copolymer | | | | | 0,1 | 0,1 | | |
| Magnesium Aluminium Silicate | 0,05 | 0,05 | 0,1 | | | | | |
| Sodium Stearoyl Glutamate | | | | | 0.05 | 0.05 | | |
| Chondrus Crispus | | | | | | 0,1 | | |
| Xanthan gum | | | | | 0,1 | | | |
| Carbomer | | | | | | | 0,2 | 0.1 |
| Hydroxypropyl Methylcellulose | | | | 0,05 | | | | |
| Glycerin | 10 | 12 | 5 | 7,5 | 5 | 5 | 7,5 | 10 |
| Panthenol | | | | | | | | 1 |
| EDTA | 1 | 1 | | 1 | 1 | | | |
| Preservative | q.s. | q.s. | q.s. | q.s. | q.s. | q.s. | q.s. | q.s. |
| Alcohol denat. | | | | | | | | 4 |
| Natriumhydroxid | q.s. | q.s. | q.s. | q.s. | q.s. | q.s. | q.s. | q.s. |
| Wasser | Ad 100 | Ad 100 | Ad 100 | Ad 100 | Ad 100 | Ad 100 | Ad 100 | Ad 100 |

### O/W Creme

| | **1** | **2** | **3** | **4** | **5** | **6** | **7** | **8** | **9** | **10** |
|---|---|---|---|---|---|---|---|---|---|---|
| Cetearath-20 | | | 2 | | | | | | | |
| Cetearyl Alkohol, PEG 40 Castor Oil, Soldium Cetearyl Sulfate | | | | | 3 | | | | | |
| Polyglyceryl-3-Distearate | | | | | | | | | | |
| Polysorbate 60 | | 1 | | 2,25 | | 2,25 | | | | |
| Polyglyceryl-3-Methyl Glucose Distearate | | 2 | | | | | | | | |
| PEG 30 stearate + Glycerylstearate | 1,5 | | 1,5 | | | | | | | |
| PEG 30 stearate | 2,25 | | | 1,8 | | | | | | |
| PEG-40 stearate | | | | | | 1,8 | 0,8 | 0,8 | | |
| Glyceryl Stearate | | | | | 0,7 | | 2,7 | 3 | | |
| Potassium Cetyl Phosphate + Hydrogenated Palm Glycerides | | | | | | | | | | 2,5 |
| Polyglyceryl-3 Distearate | | | | | | | | | 3 | |
| Lanolin Alkohol | | | | | | | | 0,5 | | |
| Stearyl Alkohol | | | | 1 | | | | 2 | | |
| Cetearyl Alkohol | | 2 | 2 | | | | 3 | 1 | 2 | 2 |
| Cetyl Alkohol | 1,5 | | | 1,5 | | | | 1 | | 0,5 |
| Paraffinum Perliquidum | | | | 2 | | | | 0,5 | | |
| Petrolatum | | | | | | | | 2 | | |
| C12-15 Alkyl Benzoate | | 1,5 | | 2 | | | 2,5 | | 3 | 2 |
| Octyldodecanol | | 3 | | | | | 2,5 | | 3 | 3 |
| Dicaprylyl Ether | 2 | | 2 | 2 | | | 2,5 | | 2 | |
| Isohexadecan | 2 | | | | | | | 1 3 | | |
| Dimethicone | 2 | 1 | 2 | | | | 1 | | 1 | 2 |
| Cyclomethicone | 2 | 2 | | 3 | | | 1 | | 2 | 2 |
| Isopropylpalmitat | | | 3 | | | | | 1 | | |
| Isohexadecan | | | | | | | | 1 | | |
| Caprylic/Capric triglyceride | | 2 | | 1 | | | | 2 | 2 | |
| Sheabutter | 1 | | 2 | | | | | | 1 | 2 |
| Avocadoöl | 0,5 | | | | | | | | | |
| Squalan | | | | 0,5 | | | | | | |
| Xanthan gum | 0,25 | | | | | | | | 0.15 | |
| Hydroxyethylcellulose | | | | | | 0,1 | | | | 0,1 |
| Carragenan | | | 0.05 | | | | | | | |
| Alkyl Acrylate Crosspolymer | 0,4 | 0,5 | 0,3 | 0,25 | 0,4 | 0,4 | | 0.4 | | 0,3 |
| Ammonium Acryloyldemethyltaurate | 0,2 | | | | | | | | | |
| Polyacrylsäure Carbomer | | | | | | | | 0,1 | | |
| Acrylate Crosspolymer | | | 0,15 | | | | 0,4 | | | |
| Butylenglycol | | | | 3 | | | 2 | | | |
| Glycerin | 5 | 5 | 10 | 5 | 10 | 7,5 | 5 | 10 | 7,5 | 8 |
| Tocopherylacetat | | 0,5 | | | | | | | | |
| Panthenol | 0,3 | | 0.2 | | | | | | | |
| 1,2 Decandiol | 0,5 | 0,5 | 0,5 | 0,5 | 0,5 | 0.5 | 0,5 | 0,5 | 0,5 | |
| Ubichinon (Q10) | | 0.05 | | | | | | | | |
| TiO₂ | | | | 1 | 1 | 1 | | | | |
| Ethylhexylmethoxycinna mat | 1,5 | | | 3 | 2 | | 3 | 5 | | |
| 2 Ethylhexyl-2-cyano-3-diphenylacrylat ( Octocrylen) | | | | | | 5 | | | | |
| Butylmethoxydibenzoyl methan | | | | | 1 | | | | | |
| Bis-Ethylhexyloxyphenolmet | | | | 1 | | | | | | |
| hoxyphenyltriazine | | | | | | | | | | |
| Natrium Ascorbylphosphat | | | | | | | | | | |
| Trinatrium EDTA | 0.1 | 0,15 | 0.2 | 0,2 | 0,15 | 0.1 | 0,15 | 0,1 | | |
| Füllstoffe (Distärkephosphat, SiO2, Talkum, Aluminiumstearat, Aluminium Starch Octenyl Succinate, Maisstärke, Reisstärke, Nylon-12, Tabiokastärke) | 2 | 3 | 1 | 4 | 1 | 3 | | | | |
| Propylparaben | 0.05 | 0.05 | 0.05 | 0,05 | 0,05 | 0,05 | 0,05 | 0,05 | 0.05 | 0.1 |
| Methylparaben | 0,1 | 0,1 | 0,2 | 0,15 | 0,15 | 0,1 | 0,1 | 0,1 | 0.1 | 0.2 |
| Phenoxyethanol | 0,4 | 0,45 | 0,3 | 0,5 | 0,4 | 0,5 | 0,5 | 0,5 | 0.5 | 0.3 |
| Natriumhydroxid | q.s. | q.s. | q.s. | q.s. | q.s. | q.s. | q.s. | q.s. | q.s. | q.s. |
| Parfuem | q.s. | q.s. | q.s, | q.s. | q.s. | q.s. | q.s. | q.s. | q.s. | q.s. |
| Wasser ad 100 | Ad 100 | Ad 100 | Ad 100 | Ad 100 | Ad 100 | Ad 100 | Ad 100 | Ad 100 | Ad 100 | Ad 100 |

## Patentansprüche

1. Kosmetische Zubereitung, **dadurch gekennzeichnet, dass** sie
a) 0,1 bis 1 Gew.-% 1,2-Decandiol und
b) 0,1 bis 0,5 Gew.-% eines oder mehrerer Acrylat-Crosspolymere
enthält.

2. Zubereitung nach Anspruch 1, **dadurch gekennzeichnet, dass** der Gehalt an 1,2-Decandiol - bezogen auf das Gesamtgewicht der Zubereitung - aus dem Bereich von 0,3 bis 0,7 Gew.-% gewählt wird.

3. Zubereitung nach Anspruch 1, **dadurch gekennzeichnet, dass** der Gehalt an 1,2-Decandiol - bezogen auf das Gesamtgewicht der Zubereitung - wie 0,5 Gew.-% gewählt wird.

4. Zubereitung nach einem der vorhergehenden Ansprüche, **dadurch gekennzeichnet, dass** das oder die Acrylat-Crosspolymere aus der Gruppe der polymere Emulgatoren gewählt wird.

5. Zubereitung nach einem der vorhergehenden Ansprüche, **dadurch gekennzeichnet, dass** das oder die Acrylat-Crosspolymere aus der Gruppe der Substanzen gewählt wird, welche unter den Handelsbezeichnungen Pemulen TR-1 und Pemulen TR-2 sowie Carbopol 1342, Carbopol 1382 und Carbopol ETD 2020 von der Firma NOVEON erhältlich sind.

6. Zubereitung nach einem der vorhergehenden Ansprüche, **dadurch gekennzeichnet, dass** der Gehalt an Acrylat-Crosspolymeren (eine oder mehrere Verbindungen) aus dem Bereich von 0,1 bis 0,5 Gew.-% gewählt wird, bezogen auf das Gesamtgewicht der Zubereitung.

7. Zubereitung nach einem der vorhergehenden Ansprüche, **dadurch gekennzeichnet, dass** sie eine Emulsion oder Hydrodispersion darstellt.

8. Zubereitung nach Anspruch 6, **dadurch gekennzeichnet, dass** sie ein feindisperses System vom Typ Öl-in-Wasser darstellt.

9. Emulsion nach einem der Ansprüche 6 oder 7, **dadurch gekennzeichnet, dass** sie einen oder mehrer O/W-Emulgatoren aus der Gruppe Glycerylstearat, PEG-40 Stearat, Triglycerinmethylglucosedistearat, Polyglyceryl-3 Methylglucose Distearate, Polyethylenglycol(21)stearylether, Polyethylenglycol(2)stearylether, Cetearylglucosid, Glycerylstearatcitrat, Natriumcetearyl-sulfat, Cetearylalkohol und/oder Sorbitanstearat enthält.

10. Zubereitung nach einem der Ansprüche 1 bis 5, **dadurch gekennzeichnet, dass** sie ein Gel darstellt und ein oder mehrere Hydrokolloide als Verdickungsmittel enthält.
